Europäisches Patentamt

European Patent Office

Office européen des brevets

(19)

(11) Publication number: **0 386 361**
**A1**

(12) # EUROPEAN PATENT APPLICATION

(21) Application number: **89310340.8**

(22) Date of filing: **10.10.89**

(51) Int. Cl.5: **A61B 17/08, A61F 9/00**

(30) Priority: **11.10.88 US 256013**

(43) Date of publication of application:
**12.09.90 Bulletin 90/37**

(84) Designated Contracting States:
**BE DE ES FR GB IT LU NL**

(71) Applicant: **ETHICON INC.**
**U.S. Route 22**
**Somerville New Jersey 08876(US)**

(72) Inventor: **Failla, Stephen J.**
**39 Dogwood Drive**
**Chester New Jersey 07930(US)**
Inventor: **Willard, Donald E.**
**1901 Hay Terrace**
**Easton Pennsylvania 18042(US)**

(74) Representative: **Fisher, Adrian John et al**
**CARPMAELS & RANSFORD 43 Bloomsbury**
**Square**
**London WC1A 2RA(GB)**

(54) **Ophthalmic staple and instruments for implementing use.**

(57) The invention generally relates to surgical staples for implanting in the eye.

FIG. I

EP 0 386 361 A1

The present invention generally relates to surgical staples for implanting in the eye. More specifically, this invention relates to surgical staples for implanting in the eye whereby the staples are adjustable while in the eye to regulate the tension exerted by the staple on the eye.

Background of the Invention

In recent years, medical science has made great steps toward improving vision in the human eye. Among these improvements, are various forms of surgery on a living eye. One of the more common forms of surgery is the removal of a cataractus lens, which generally takes the form of a hardened lens tissue. It is this cataractus tissue which will cause the blurring of vision. Frequently, of course, cataracts will appear in the eyes of the elderly.

In order to remove cataractus tissue, generally the lens will be removed and replaced with an intraocular lens. This must be done through an incision made with a scalpel in the eye. This incision is generally formed in the eye at the limbus, which is generally the area where the clear cornea meets the more whiteish-colored sclera. After a linear incision has been made, various instruments can be placed within the eye in order to remove the cataractus lens. Sometimes, a phacoemulsification instrument will be used. After the instrument has removed the cataractus lens from the eye, and the intraocular lens emplaced within the eye, the incision made in the eye must be closed. This closing of the incision will present various problems. For instance, if the incision is closed too tightly, the resultant healing of the eye will cause a generally "with the rule" astigmatism. On the other hand, it is also possible that the incision is closed too loosely. This results in what is known as "against the rule" astigmatism. Either type of astigmatism can readily result in about ± 1 to 2 diopters of change in vision after surgery.

Generally, when sutures are used, the surgeon will keep these sutures tighter than required to close the incision. This results in a controlled amount of "with the rule" astigmatism. After surgery, some sutures can be cut in order to reduce the astigmatism to improve vision to be near normal. On the other hand, if the sutures are too loose, it is virtually impossible without re-operation to correct the "against the rule" astigmatism after surgery has been completed

In addition, sutures are sometimes adjustable during operation by using a slip knot in the sutures. This slip knot can be adjusted so that proper tension is applied to the wound. Of course, this adjustment of the sutures by a slip knot is difficult to implement, and requires much training and expertise.

The reason for introducing an initial "with-the-rule" astigmatism is that it degrades with time, ideally to no astigmatism. However, further degradation will result in "against-the-rule" astigmatism. This degradation is caused by a combination of two factors -- postoperative stretching of the suture and "cheese knifing" of the suture through some of the eye tissue within the suture loop.

With this astigmatism problem, as well as the problem of keeping incision lengths small in order to avoid contamination and/or trauma to the open site, it becomes extremely important to find an accurate, reliable and safe way to close medical cuts made in the eye surface. The use of staples is indicated.

On the other hand, staples have been difficult to implement because of the general form of a staple. For instance, most surgical skin staples are rigidly box shaped. This box shape is not adjustable while in the eye either during operation or postoperatively because of its need to be pressured against the eye during forming. Also because of the small tolerances within the eye, precise and delicate stapling systems are required to implant the staples.

In addition, removal of these staples is difficult because the staple should generally be removed through its insertion point. If a box shaped staple is removed it will generally rip tissue on its way out, which is undesirable. The same holds true for a "B" shaped staple. There, an even greater tissue tear takes place. What is necessary, therefore, is a staple which is able to be removed from precisely the same path in which it enters the tissue, or a staple which can be emplaced within the eye and cause minimal trauma, so that it can remain in the eye.

Summary of the Invention

It is therefore an object of the present invention to provide a surgical staple emplaceable within the eye. The surgical staple should be easily emplaceable and capable of controlling the amount of tension created on the eye surface. In addition, it is an object of the present invention to provide a surgical staple that will remain in the eye after surgery.

It is a further object of the present invention to provide a surgical staple in which the tension the staple exerts on the eye is adjustable while the staple is in the eye.

It is yet another object of the present invention to provide a surgical staple which will not cause a large amount of tissue trauma when emplaced within the eye.

It is a further object of the present invention to be able to apply a surgical staple in the eye with forceps, needle holders, tweezers and/or similar specially formed applying instruments, instead of the conventional surgical staplers using drivers and the like.

It is yet another object of the present invention to provide a surgical staple which is implantable and adjustable in the eye with a pair of specially formed pliers and in which the tension can be adjusted while the staple is implanted within the eye.

It is finally an object of the present invention to provide a surgical staple in which the staple is insertable into the eye by inserting one leg into the eye, on one side of the incision, then stretching the crown of the staple, inserting the second leg of the staple into the eye on the second side of the incision, and then adjusting the entire staple in order to create the appropriate tension on the eye.

These and other objects of the present invention are accomplished in a surgical staple which has two legs which are joined by a generally resilient crown. The legs of the staple are situated in a plane perpendicular to the crown. The crown contains a spring-like member which can be adjusted, so that the legs of the staple are movable relative to one another by deformation of the spring-like member. In addition, the staple is insertable within tissue such as the tissue of the eye by use of a forceps or needle holder-like device which is able to grip the staple. One piercing leg is inserted; the staple is stretched to draw the incision closed; the second staple leg is then inserted into the far side of the incision; the staple is then adjusted for appropriate tension on the eye. The adjusting device is generally pliers-shaped with mushroom shaped heads, and insertable within the spring-like portion of the crown.

## Brief Description of the Drawings

The invention will be more fully described in the following detailed description of the invention in conjunction with the accompanying drawings wherein:

Fig. 1 is a perspective view of a preferred embodiment of the staple of the present invention.

Fig. 2a is a top plan view of a preferred embodiment of the present invention.

Fig. 2b is an elevation view of a preferred embodiment of the present invention.

Fig. 3a is a top plan view of a second preferred embodiment of the present invention.

Fig. 3b is an elevation view of a second preferred embodiment of the present invention.

Fig. 4a is a top plan view of a third preferred embodiment of the present invention.

Fig. 4b is an elevation view of a third preferred embodiment of the present invention.

Fig. 5a is a top plan view of a fourth preferred embodiment of the present invention.

Fig. 5b is an elevation view of a fourth preferred embodiment of the present invention.

Fig. 6a is a top plan view of a fifth preferred embodiment of the present invention.

Fig. 6b is an elevation view of a fifth preferred embodiment of the present invention.

Fig. 6c is a top plan view of a sixth preferred embodiment of the present invention.

Fig. 6d is an elevation view in partial cross section of a sixth preferred embodiment of the present invention.

Fig. 7 is a perspective view of the application instrument of the present invention inserting the staple of the present invention into tissue.

Fig. 8a, Fig. 8b and Fig. 8c are elevation views of the insertion of the staple of the present invention into tissue.

Fig. 9 is a partial elevation view of a leg of the staple of the present invention.

Fig. 10 is a perspective view of a preferred embodiment of an adjusting tool of the present invention.

Fig. 10a is a perspective view of the heads of an adjusting tool of a second preferred embodiment of the present invention.

Fig. 11a and Fig.11b are perspective views of tensioning and removal of the staple of the present invention by using an adjusting tool of the present invention.

Fig. 12 is a perspective view of adjusting staple tension in the staple of the present invention.

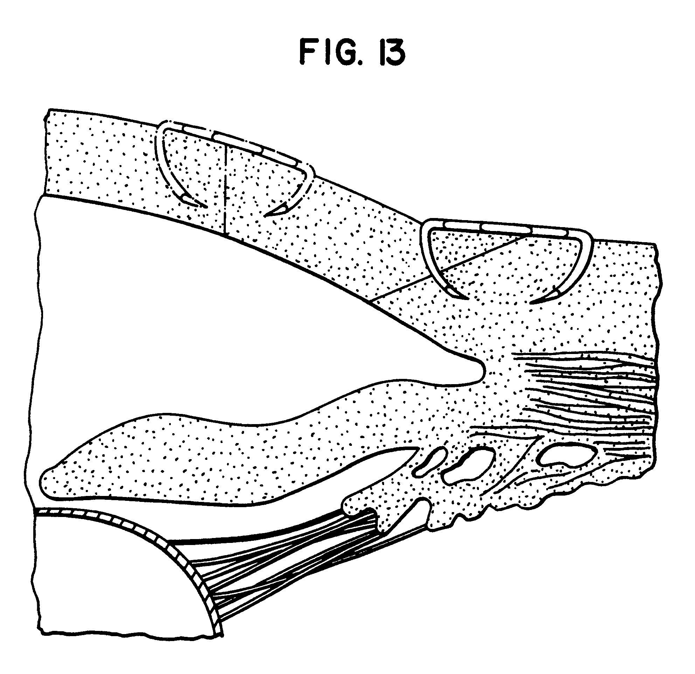

Fig. 13 is a top plan view of the staple of the present invention as inserted into a limbal incision in the eye.

## Detailed Description of the Present Invention

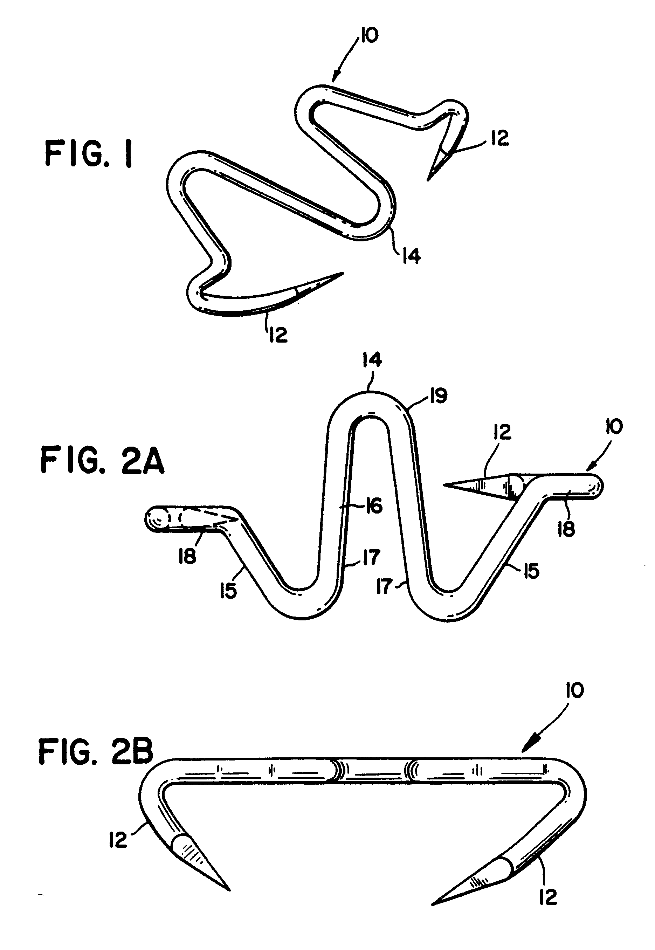

As can be seen in Figures 1, 2a, 2b, 3a, 3b, 4a, 4b, 5a, 5b, 6a, 6b, 6c and 6d, ophthalmic staples 10, 20, 30, 40, 50 and 60 disclose various preferred embodiments of the present invention. As more accurately disclosed in Figure 1, with each of these ophthalmic staples there will be a pair of legs 12 and a crown 14. The crown 14 lies in a plane generally perpendicular to the plane of the legs as can be seen from Figure 2a and 2b. The crown 14 is spring-like so that it allows the relative motion of the legs 12 with one another, by deflection of the crown 14. This is generally due to the resilient spring-like member 16 of the crown 14 which is able to be deflected depending on the desired tension in the staple 10. This spring-like member 16 joins rigid members of the crown 18. These rigid members 18 connect the generally piercing legs 12 to the crown 14.

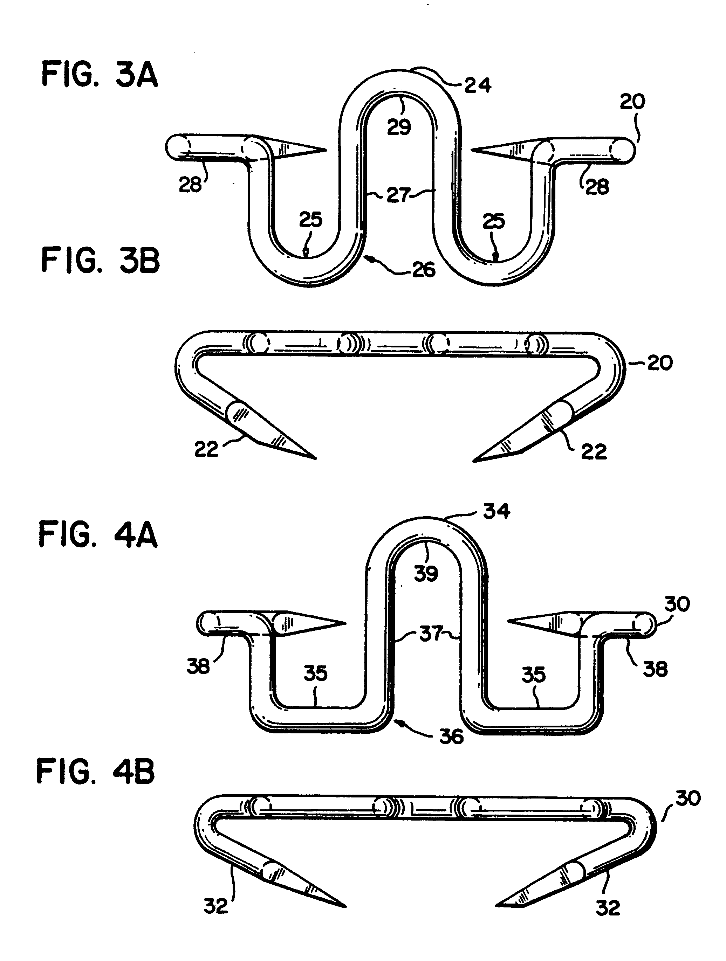

Generally, the spring-like member 16 will contain two cross members 17 which are connected by a generally flexible connecting member 19. At their opposite ends, the generally rigid cross members 17 are connected to a receiving member 15. This receiving member 15 is, in turn, connected to the generally rigid members 18 of the crown portion 14. The use of these receiving members 15 will be explained shortly. Thus, as can be seen in Figure 3 and 3a, there is an ophthalmic staple 20 having legs 22, a crown 24, a resilient spring-like member 26, rigid members 28, cross-members 27, flexible connecting members 29 and rounded receiving members 25, all corresponding to the members in ophthalmic staple 10. This staple takes on a more rounded shape in order to provide greater flexibility to the staple.

As seen in Figures 4a and 4b, the corresponding ophthalmic staple 30 contains legs 32, crown 34, spring-like member 36, rigid member 38, cross member 37, and flexible connecting member 39. Receiving member 35 has been made straight instead of having a rounded appearance. This straight receiving member 35 is useful in minimizing the width of the staple.

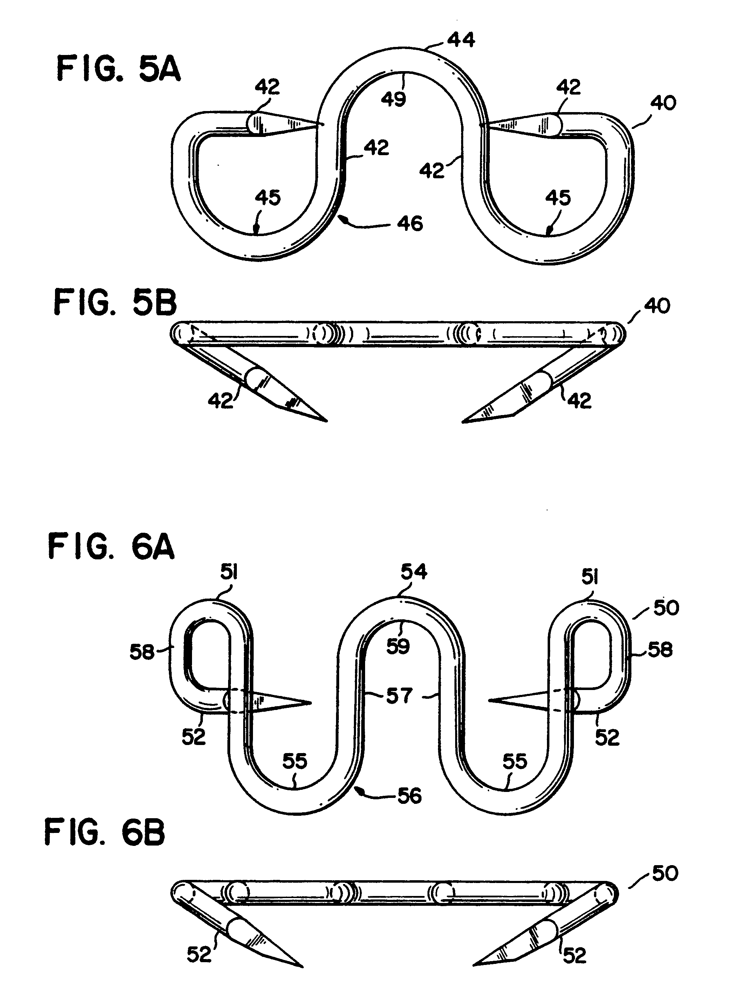

On the other hand, Figures 5a and 5b disclose ophthalmic staple 40 which contains legs 42, crown 44, spring-like member 46, cross member 47, flexible connecting member 49 and rounded receiving member 45. In this type staple there is no additional rigid member attached to the legs of the staple. This, of course, reduces the total length of the staple.

On the other hand, where more rigidity is desired in the positioning of the legs, one may use ophthalmic staple 50, as seen in Figures 6a and 6b. In this device there are legs 52, crown 54, spring-like member 56, rigid member 58, cross member 57, flexible connecting member 59 and rounded receiving member 55, all having a form generally corresponding to staple 10. In addition, however, there are provided loops 51 useful in the placement of the legs without relying excessively on deflection of the spring-like member 56. These loops 51 are connected to a rigid member 58 which is now generally perpendicular to the legs 52 in the plane of the crown 54. This allows for a greater control of the lateral, or side-to-side motion of the staple 50 while easily ensuring accurate in-tissue placement of the staple 50.

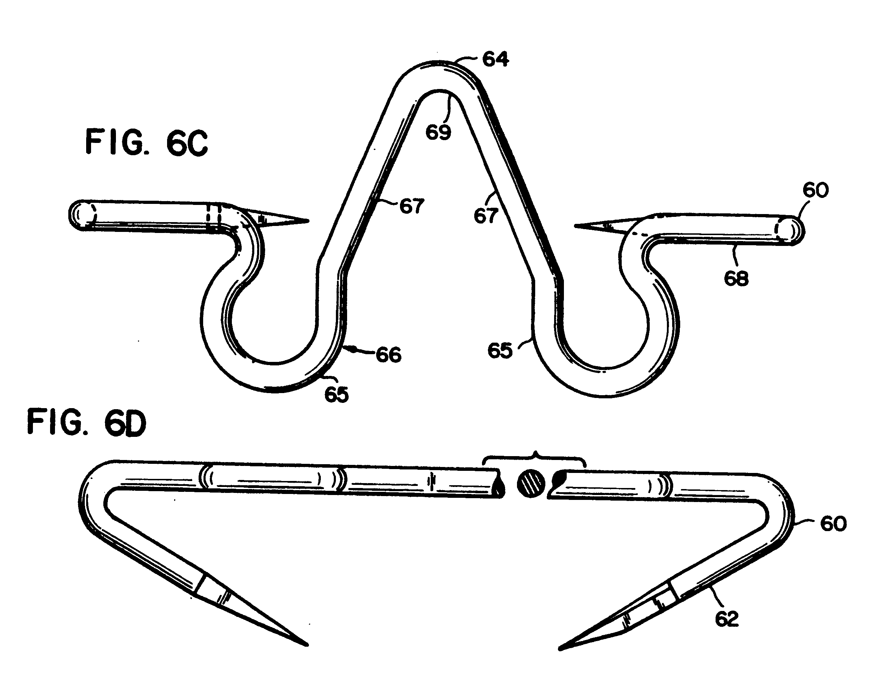

Ultimately, the staple of the present invention will take on the generally preferred embodiment of staple 60 demonstrated in Figures 6c and 6d. In this staple 60, there are legs 62 which generally extend at about 30° angles from the crown 64 of the staple. As in all previously demonstrated ophthalmic staples 10, 20, 30, 40, 50, the plane of the legs 62 is perpendicular to the plane of the crown 64. The crown 64 contains a spring-like member 66 which is comprised of a flexible connecting member 69 and rigid cross members 67. These cross members 67 extend at about 60° angles away from the peak at the flexible connecting member 69. The cross members 67 are connected at their other ends to rounded receiving members 65. In addition, the rounded receiving members 65 describe nearly an entire circle before attachment to rigid members 68, attached to the legs 62.

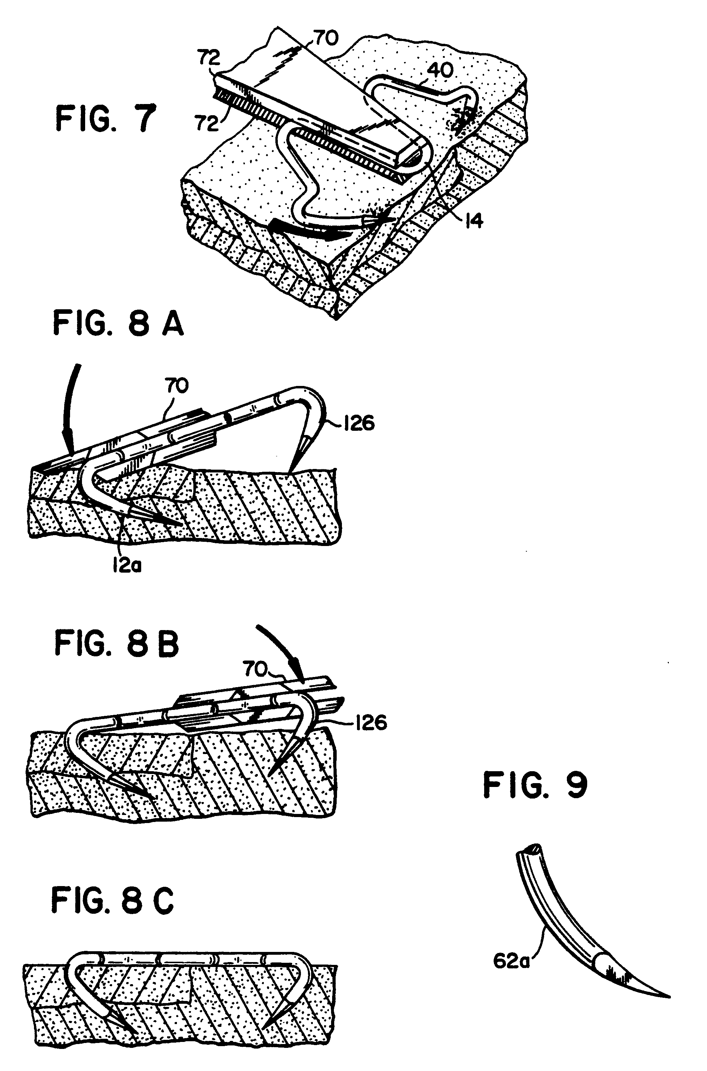

In general, this staple has a diameter from $.0015''$ to $.005''$, generally about $.003''$, and the length of the staple at the crown is $.040''$ to $.120''$, generally about $.080''$. The staple legs are roughly $.015''$ to $.050''$ in length, generally about $.028''$; the legs extend at angles of between 20° and 55° to the plane of the crown usually about 30°. The width of the staple is about $.010''$ to $.080''$, usually equally divided on both sides of the plane of the legs. The legs can either be straight (legs 62 of Figure 6d) or rounded (legs 62a of Figure 9).

When placement of these staples is desired, the steps in Figure 7 and Figures 8a, 8b and 8c are followed. A generally tweezer-like instrument 70 having legs 72 grasps the staple 10. The grasp is such that the plane of the tweezer-like instrument lies in the same plane as the crown 14. First, one leg is embedded into the tissue of the eye as seen in Figure 8a. This is accomplished by grasping the tweezer-like instrument 70 near the end of the crown 14 toward first leg 12a. At that point, the tweezer-like instrument 70 is shifted to the other side in order to insert leg 12b. Intermediate this step the crown 14 is stretched elastically in order to properly embed both the legs, 12a and 12b. This will avoid deforming the legs when inserting leg 12b.

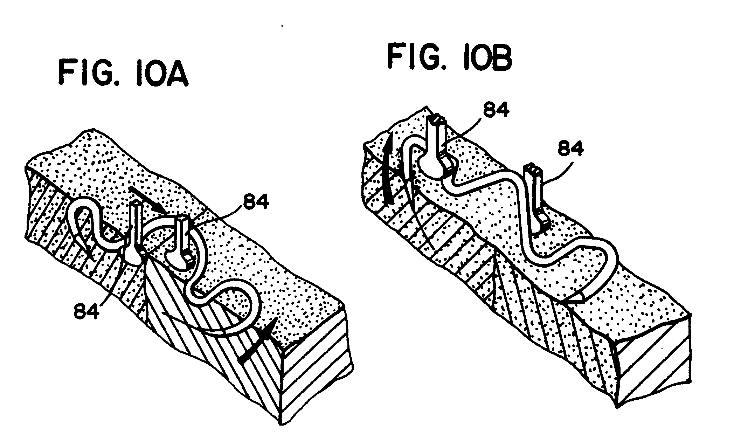

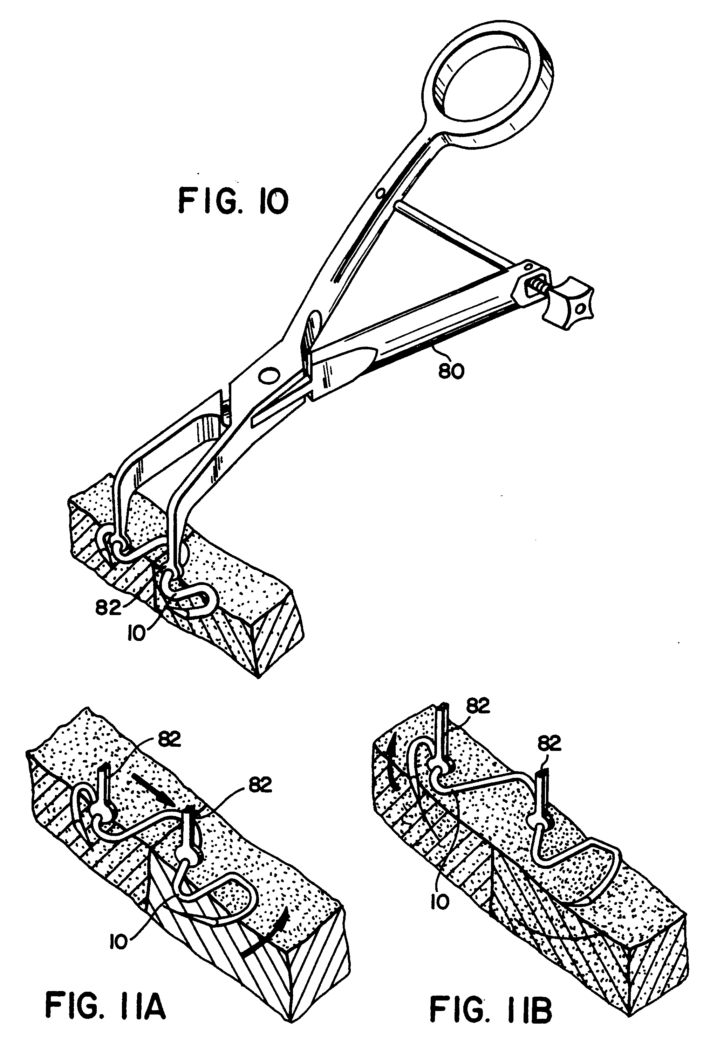

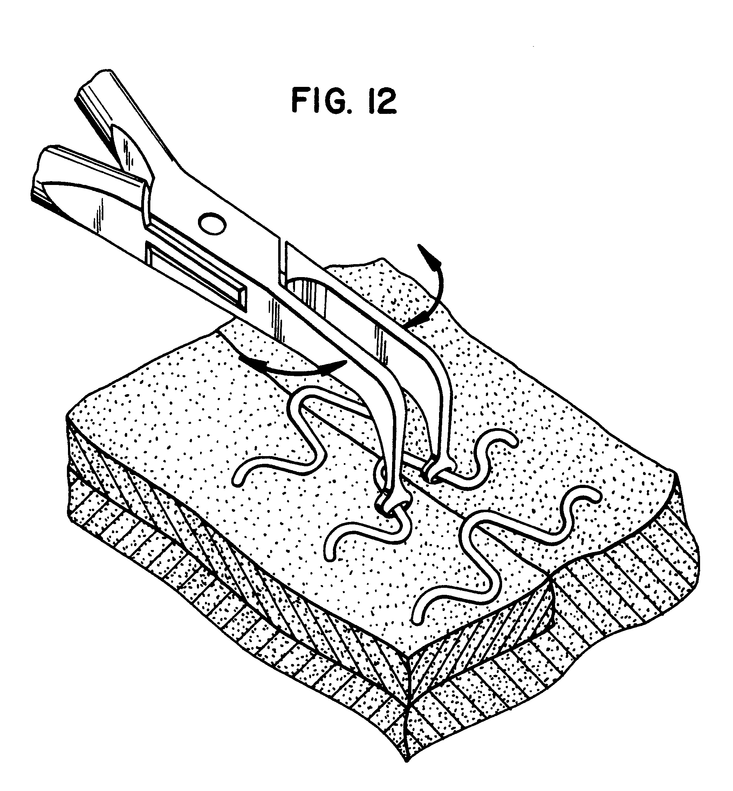

At this point, the staple is ready for adjustment. Generally, a pliers-like tool 80 as seen in Figure 10 is used for adjustment. This pliers-like tool contains grasping prongs 82 which fit around the legs. As seen in Figures 11a and 11b, or Figure 12, these grasping prongs 82 can be used to adjust the tension in the ophthalmic staple. By having the grasping prongs pull the staple together, the tension the legs 12 exert on the tissue can be increased. By pulling the legs apart, the tension that legs 12 exert on the tissue can be decreased. In fact, as seen in Figure 11b, the staple 10 can be removed without trauma to the eye.

As seen in Figures 10a and 10b, the grasping prongs 82 are modified to take on the mushroom shape of prongs 84. These mushroom shaped prongs 84 are more preferable than the grasping prongs in that they need not be affixed around the extremely small staple 10. In fact, the mushroom shaped prongs 84 generally fit into the rounded receivers such as the rounded receiver 65 seen in Figure 6. These mushroom shaped prongs 84 can also ease or exert more tension on the staple 10.

Generally, the staples are implanted at the end of ophthalmic surgery. Usually, the conjunctiva is refracted from the sclera. The staple is placed on the sclera surface. Generally, the staple is then left permanently in the eye. The conjuctiva is pulled over the staple and the staple becomes embedded in the sclera. The conjuctiva heals quickly, usually within two to three days. There is generally no worry about conjunctivitis because the staple is formed from an implantable metal, often stainless steel or vitalium. When the staple becomes embedded in the sclera, the conjuncfiva heals smoothly over the staple, preventing trauma.

While the staple is permanently embedded in the sclera, it can still be adjustable. If it desired to increase the tension on the staple, the mushroom shaped prongs 84 of the pliers-like instrument 80 are placed within the conjunctiva and within the rounded receiving member 65. The staple is eased together to increase or decrease the tension exerted by the legs 12 on the tissue. Alternately, mushroom shaped prong 84 can relieve the tension in the staple 10 by easing the crown apart so that the legs 12 are relaxed within the sclera.

The unique combination of features possessed by the present invention render them suited for in the eye implantation. These combinations may be useful for other forms of tissue implantation outside the eye. Naturally, while several means are available, the particularly advantageous embodiments have been chosen to illustrate the invention. It will be understood by those skilled in the art that various changes and modifications may be made therein without departing from the scope of the invention, which is defined by the following claims and their equivalents.

## Claims

1. A tissue staple comprising two legs, said legs joined by an adjustable crown, said legs and said crown laying in generally perpendicular planes, said crown containing a spring-like member wherein the legs are movable relative to one another by deflection of said spring-like member.

2. A tissue staple comprising two legs connected by a crown lying in a plane perpendicular to the plane of said legs, said crown having a an adjustable portion capable of expanding and contracting in tissue, effecting movement of said legs relative to each other.

3. A tissue staple comprising two legs adapted for piercing tissue in order to gather tissue between said legs, said legs connected by a generally adjustable crown lying in a plane generally perpendicular to said legs, said crown comprising two generally rigid members each connected to one of said legs, said rigid members joined by a generally spring-like member, capable of expansion and contraction in the plane of said crown in order to cause said legs to expand and contract in the plane of said legs.

4. The staple of Claim 3 wherein the plane of said crown is adapted to lie on the surface of said tissue when said staple is emplaced in said tissue.

5. The staple of Claim 4 wherein said spring-like member comprises a pair of cross members joined by a generally flexible connecting member, said cross members each connected at their free ends to said generally rigid members by a generally rounded spring-like receiving member.

6. The staple of Claim 5 wherein each said receiving member is adapted to receive the prongs of an adjusting tool in order to adjust the position of said legs by effecting tension on said spring-like member.

7. The staple of Claim 4 wherein said legs are generally curved to gather said tissue between said curved legs.

8. The staple of Claim 4 wherein said legs are generally straight and pierce said tissue at oblique angles such that tissue is gathered between said legs.

9. A tool for emplacement of staples having a crown joining two legs, said crown lying in a plane generally perpendicular to said legs, comprising a generally flat gripping portion capable of holding said crown while leaving said legs free for emplacement into tissue.

10. The tool for emplacement of staples of Claim 9 wherein said crown has generally parallel cross members lying in the plane of said crown, said tool gripping portion lying between said cross members and gripping said cross members.

11. A tool for adjusting a staple having a generally adjustable crown joining two legs, said crown lying in a plane generally perpendicular to said legs and containing a spring-like member having generally curved receiving members movable relative to one another, said tool comprising a pliers-like instrument having two ends capable of emplacement within said curved receiving ends, in order to effect their relative motion and allow the adjustment of said legs into tissue.

12. The tool for adjustment of staples of Claim 11 wherein said ends of said tool are each mushroom shaped in order to be attached to the rounded receiving members in order to move said crown and said legs relative to one another.

FIG. 1

FIG. 2A

FIG. 2B

FIG. 3A

FIG. 3B

FIG. 4A

FIG. 4B

FIG. 5A

FIG. 5B

FIG. 6A

FIG. 6B

FIG. 6C

64

69

67    67

60

66

68

65

65

FIG. 6D

60

62

EP 0 386 361 A1

FIG. 7

FIG. 8 A

FIG. 8 B

FIG. 9

FIG. 8 C

FIG. IOA

FIG. IOB

FIG. 10

80

82

10

FIG. 11A

82

82

10

FIG. 11B

82

82

10

# FIG. 12

FIG. 13

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-3 586 002 (WOOD) * Entire document * | 1-7 | A 61 B 17/08 A 61 F 9/00 |
| X | US-A-2 684 070 (KELSEY) * Entire document * | 1-8 | |
| X | US-A-2 817 339 (SULLIVAN) * Figure 8; column 4, lines 1-12 * | 9 | |
| X | US-A-4 217 902 (MARCH) * Figures 3,6; column 2, line 43 - column 3, line 22 * | 11 | |
| Y | | 12 | |
| Y | DE-A-3 337 447 (FRYDRYCH) * Page 5, lines 11-12; claim 1; figure 1 * | 12 | |
| A | DE-C- 617 447 (SAMUEL) | | |
| A | US-A-3 209 753 (HAWKINS) | | TECHNICAL FIELDS SEARCHED (Int. Cl.5) |
| A | WO-A-8 801 955 (OPHTHALMIC VENTURES) | | A 61 B A 61 F |
| P,X | WO-A-8 904 146 (OPHTHALMIC VENTURES) * Abstract; Figures * | 1 | |

The present search report has been drawn up for all claims

| Place of search | Date of completion of the search | Examiner |
|---|---|---|
| THE HAGUE | 19-01-1990 | STEENBAKKER J. |